(19) Europäisches Patentamt / European Patent Office / Office européen des brevets

(11) EP 4 071 470 A1

(12) **EUROPEAN PATENT APPLICATION**
published in accordance with Art. 153(4) EPC

(43) Date of publication:
**12.10.2022 Bulletin 2022/41**

(21) Application number: **20896839.6**

(22) Date of filing: **02.12.2020**

(51) International Patent Classification (IPC):
**G01N 33/48** (2006.01)

(52) Cooperative Patent Classification (CPC):
**G01N 33/48**

(86) International application number:
**PCT/JP2020/044835**

(87) International publication number:
**WO 2021/112119 (10.06.2021 Gazette 2021/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.12.2019 JP 2019220497**

(71) Applicants:
• **Sekisui Medical Co., Ltd.**
**Tokyo 103-0027 (JP)**

• **Tokuyama Sekisui Co., Ltd.**
**Osaka-Shi, Osaka 530-8565 (JP)**

(72) Inventor: **KOMAI, Kuniya**
**Shunan-shi, Yamaguchi 746-0006 (JP)**

(74) Representative: **Ter Meer Steinmeister & Partner Patentanwälte mbB**
**Nymphenburger Straße 4**
**80335 München (DE)**

(54) **BLOOD COLLECTION CONTAINER AND PLASMA SEPARATION METHOD**

(57) There is provided a blood collection container capable of suppressing the contamination of white blood cells into blood plasma. A blood collection container according to the present invention is a blood collection container into which a predetermined amount of blood is collected. The blood collection container includes a blood collection container main body, and a blood plasma separation material, an osmotic pressure regulator, and an anticoagulant contained in the blood collection container main body. The specific gravity of the blood plasma separation material at 25°C is 1.030 or more and 1.120 or less. When the osmotic pressure regulator and the anticoagulant contained in the blood collection container main body are dissolved in an amount of physiological saline solution equivalent to a predetermined amount of blood collected in the blood collection container to obtain an osmotic pressure measurement solution, the specific gravity of the blood plasma separation material at 25°C and the osmotic pressure of the osmotic pressure measurement solution satisfy a specific relationship.

[FIG. 1]

**Description**

**TECHNICAL FIELD**

The present invention relates to a blood collection container. The present invention also relates to a method for separating blood plasma using the blood collection container.

**BACKGROUND ART**

In clinical examinations, a blood collection container such as a blood collection tube is widely used to collect blood. After the blood is collected in the blood collection container containing a blood plasma separation material, the blood can be separated into blood plasma and blood cells by centrifuging the blood collection container. At this time, the blood plasma is located on the upper side of the blood plasma separation material, and the blood cells are located on the lower side of the blood plasma separation material. As a blood collection container containing a blood plasma separation material, there are known a blood collection container containing a blood plasma separation composition containing a resin, an inorganic powder, and the like (e.g., Patent Document 1), and a blood collection container containing a blood plasma separation jig (e.g., Patent Document 2).

**Related Art Document**

**Patent Document**

Patent Document 1: WO 2010/053180 A1
Patent Document 2: WO 2010/132783 A1

**SUMMARY OF THE INVENTION**

**PROBLEMS TO BE SOLVED BY THE INVENTION**

In clinical examinations, examinations using blood plasma are performed. When blood plasma is separated from blood using a conventional blood collection container, white blood cells may be contaminated in the separated blood plasma. When white blood cells are contaminated in the blood plasma, the white blood cells may be broken, and components such as proteins and nucleic acids in the white blood cells may leak into the blood plasma, affecting the test result.

For example, in a test for detecting extracellular nucleic acid (e.g., cell free DNA) in blood plasma, the test result greatly varies depending on nucleic acid leaking from white blood cells.

An object of the present invention is to provide a blood collection container capable of suppressing the contamination of white blood cells into blood plasma. Another object of the present invention is to provide a method for separating blood plasma using the blood collection container.

**MEANS FOR SOLVING THE PROBLEM**

According to a broad aspect of the present invention, there is provided a blood collection container into which a predetermined amount of blood is collected, the blood collection container including: a blood collection container main body; a blood plasma separation material contained in the blood collection container main body; an osmotic pressure regulator contained in the blood collection container main body; and an anticoagulant contained in the blood collection container main body, in which a specific gravity of the blood plasma separation material at 25°C is 1.030 or more and 1.120 or less, and when the osmotic pressure regulator and the anticoagulant contained in the blood collection container main body are dissolved in physiological saline solution in an amount equivalent to a predetermined amount of blood collected in the blood collection container to obtain an osmotic pressure measurement solution, when the specific gravity of the blood plasma separation material at 25°C is 1.030 or more and less than 1.040, an osmotic pressure of the osmotic pressure measurement solution is 300 mOsm/L or more, when the specific gravity of the blood plasma separation material at 25°C is 1.040 or more and less than 1.050, the osmotic pressure of the osmotic pressure measurement solution is 330 mOsm/L or more, when the specific gravity of the blood plasma separation material at 25°C is 1.050 or more and less than 1.060, the osmotic pressure of the osmotic pressure measurement solution is 350 mOsm/L or more, when the specific gravity of the blood plasma separation material at 25°C is 1.060 or more and less than 1.070, the osmotic pressure of the osmotic pressure measurement solution is 500 mOsm/L or more, and when the specific gravity of the blood plasma separation material at 25°C is 1.070 or more and 1.120 or less, the osmotic pressure of the osmotic pressure measurement solution is 650 mOsm/L or more.

In a specific aspect of the blood collection container according to the present invention, the blood plasma separation material is a blood plasma separation composition.

In a specific aspect of the blood collection container according to the present invention, the blood plasma separation composition contains an organic component having fluidity at 25°C and an inorganic fine powder, the organic component contains a resin, and the inorganic fine powder contains fine powder silica.

In a specific aspect of the blood collection container according to the present invention, the fine powder silica contains hydrophilic silica.

In a specific aspect of the blood collection container according to the present invention, a content of the hydrophilic silica is 0.01 wt% or more and 2.50 wt% or less in 100 wt% of the blood plasma separation composition.

In a specific aspect of the blood collection container according to the present invention, the fine powder silica contains hydrophilic silica and hydrophobic silica.

In a specific aspect of the blood collection container according to the present invention, when the specific gravity of the blood plasma separation composition at 25°C is 1.05 or more, the inorganic fine powder contains an inorganic fine powder having a specific gravity larger than a specific gravity of the fine powder silica.

In a specific aspect of the blood collection container according to the present invention, the resin includes a petroleum resin, a cyclopentadiene-based resin, a polyester resin, or a (meth)acrylic-based resin.

In a specific aspect of the blood collection container according to the present invention, the osmotic pressure regulator is contained in the blood collection container main body in a powder state or in a state of being dissolved in a liquid, and the anticoagulant is contained in the blood collection container main body in a powder state or in a state of being dissolved in a liquid.

In a specific aspect of the blood collection container according to the present invention, the osmotic pressure regulator is disposed on an inner wall surface of the blood collection container main body or on a surface of the blood plasma separation material, and the anticoagulant is disposed on an inner wall surface of the blood collection container main body or on a surface of the blood plasma separation material.

In a specific aspect of the blood collection container according to the present invention, the osmotic pressure regulator is sodium chloride or glucose.

In a specific aspect of the blood collection container according to the present invention, the blood collection container is suitably used for detecting extracellular nucleic acid in blood.

According to a broad aspect of the present invention, there is provided a method for separating blood plasma using the above-described blood collection container, the method including the steps of: collecting blood in the blood collection container; and centrifuging the blood collection container into which the blood is collected.

**EFFECT OF THE INVENTION**

A blood collection container according to the present invention is a blood collection container into which a predetermined amount of blood is collected, and includes a blood collection container main body, a blood plasma separation material contained in the blood collection container main body, an osmotic pressure regulator contained in the blood collection container main body, and an anticoagulant contained in the blood collection container main body. In the blood collection container according to the present invention, the specific gravity of the blood plasma separation material at 25°C is 1.030 or more and 1.120 or less. In the blood collection container according to the present invention, when the osmotic pressure regulator and the anticoagulant contained in the blood collection container main body are dissolved in physiological saline solution in an amount equivalent to a predetermined amount of blood collected in the blood collection container to obtain an osmotic pressure measurement solution, the following configurations (1) to (5) are satisfied. (1) When the specific gravity of the blood plasma separation material at 25°C is 1.030 or more and less than 1.040, the osmotic pressure of the osmotic pressure measurement solution is 300 mOsm/L or more. (2) When the specific gravity of the blood plasma separation material at 25°C is 1.040 or more and less than 1.050, the osmotic pressure of the osmotic pressure measurement solution is 330 mOsm/L or more. (3) When the specific gravity of the blood plasma separation material at 25°C is 1.050 or more and less than 1.060, the osmotic pressure of the osmotic pressure measurement solution is 350 mOsm/L or more. (4) When the specific gravity of the blood plasma separation material at 25°C is 1.060 or more and less than 1.070, the osmotic pressure of the osmotic pressure measurement solution is 500 mOsm/L or more. (5) When the specific gravity of the blood plasma separation material at 25°C is 1.070 or more and 1.120 or less, the osmotic pressure of the osmotic pressure measurement solution is 650 mOsm/L or more. The blood collection container according to the present invention is provided with the above-described configuration, and thus it is possible to suppress the contamination of white blood cells into blood plasma.

**BRIEF DESCRIPTION OF DRAWINGS**

[Fig. 1] Fig. 1 is a front cross-sectional view of a blood collection container according to a first embodiment of the present invention.
[Fig. 2] Fig. 2 is a front cross-sectional view of a blood collection container according to a second embodiment of the present invention.

**MODES FOR CARRYING OUT THE INVENTION**

In the following, the present invention will be described in detail.

A blood collection container according to the present invention is a blood collection container into which a predetermined amount of blood is collected, and includes a blood collection container main body, a blood plasma separation material contained in the blood collection container main body, an osmotic pressure regulator contained in the blood collection container main body, and an anticoagulant contained in the blood collection container main body. In the blood collection container according to the present invention, the specific gravity of the blood plasma separation material at 25°C is 1.030 or more and 1.120 or less. In the blood collection container according to the present invention, when the osmotic pressure regulator and the anticoagulant contained in the blood collection container main body are dissolved in physiological saline solution in an amount equivalent to a predetermined amount of blood collected in the blood collection container to obtain an osmotic pressure measurement solution, the following configurations (1) to (5) are satisfied.

(1) When the specific gravity of the blood plasma separation material at 25°C is 1.030 or more and less than 1.040, the osmotic pressure of the osmotic pressure measurement solution is 300 mOsm/L or more.
(2) When the specific gravity of the blood plasma separation material at 25°C is 1.040 or more and less than 1.050, the osmotic pressure of the osmotic pressure measurement solution is 330 mOsm/L or more.
(3) When the specific gravity of the blood plasma separation material at 25°C is 1.050 or more and less than 1.060, the osmotic pressure of the osmotic pressure measurement solution is 350 mOsm/L or more.
(4) When the specific gravity of the blood plasma separation material at 25°C is 1.060 or more and less than 1.070, the osmotic pressure of the osmotic pressure measurement solution is 500 mOsm/L or more.
(5) When the specific gravity of the blood plasma separation material at 25°C is 1.070 or more and 1.120 or less, the osmotic pressure of the osmotic pressure measurement solution is 650 mOsm/L or more.

The blood collection container according to the present invention is provided with the above-described configuration, and thus it is possible to suppress the contamination of white blood cells into blood plasma.

In addition, since the blood collection container according to the present invention is provided with the configuration, it is also possible to suppress the contamination of red blood cells into blood plasma.

When blood plasma is separated from blood using a conventional blood collection container, white blood cells may be contaminated in the separated blood plasma. When white blood cells are contaminated in the blood plasma, components in the white blood cells may leak into the blood plasma, affecting the examination of the blood plasma. In a conventional blood collection container, it is difficult to sufficiently suppress the contamination of white blood cells into blood plasma. Note that in order to suppress the influence on the test result, although a blood collection container containing a cell stabilizer that stabilizes blood cells may be used, the cell stabilizer is expensive, and depending on the type and concentration, it may be harmful to human bodies and environments.

On the other hand, in the blood collection container according to the present invention, it is possible to suppress the contamination of white blood cells into blood plasma as compared with the conventional blood collection container. When blood is collected into the blood collection container according to the present invention, the osmotic pressure regulator, the anticoagulant, and the like are dissolved in the blood, and the osmotic pressure of the blood increases. Therefore, the moisture in the white blood cells and the moisture in the red blood cells move to the outside of the blood cells, and the specific gravities of the white blood cells and the red blood cells increase. The white blood cells and the red blood cells having increased specific gravity move downward better than the blood plasma separation material having a particular specific gravity by centrifuging the blood collection container. As a result, it is possible to suppress the contamination of white blood cells and red blood cells into blood plasma.

In the present invention, the osmotic pressure regulator and the anticoagulant contained in the blood collection container main body are dissolved in physiological saline solution in an amount equivalent to a predetermined amount of blood collected in the blood collection container to obtain an osmotic pressure measurement solution, and the osmotic pressure of the obtained osmotic pressure measurement solution is measured.

Specifically, the osmotic pressure measurement solution is prepared as follows.

An amount of physiological saline solution equivalent to a predetermined amount of blood collected in the blood collection container is added to the blood collection container. For example, in a blood collection container in which 5 mL of blood is collected, 5 mL of physiological saline solution is added to the blood collection container. After the addition, the mixture is mixed by inversion, and the osmotic pressure regulator and the anticoagulant are dissolved in physiological saline solution. Note that when the blood collection container contains components other than the osmotic pressure regulator and the anticoagulant that can be dissolved in physiological saline solution, the other components are also dissolved in physiological saline solution. In this manner, an osmotic pressure measurement solution can be obtained.

The osmotic pressure of the osmotic pressure measurement solution is measured by a cryoscopic method using an osmometer (e.g., "OM-6060" manufactured by ARKRAY, Inc.).

When the specific gravity of the blood plasma separation material at 25°C is 1.030 or more and less than 1.040, the osmotic pressure of the osmotic pressure measurement solution is 300 mOsm/L or more, preferably 320 mOsm/L or more, and more preferably 350 mOsm/L or more. When the osmotic pressure is the lower limit or more, the specific gravities of white blood cells and red blood cells can be effectively increased, and the contamination of white blood cells and red blood cells into blood plasma can be more effectively suppressed. Note that the upper limit of the osmotic pressure of the osmotic pressure measurement solution when the specific gravity of the blood plasma separation material at 25°C is 1.030 or more and less than 1.040 is not particularly limited. When the specific gravity of the blood plasma separation material at 25°C is 1.030 or more and less than 1.040, the osmotic pressure of the osmotic pressure measurement solution may be, for example, 1,500 mOsm/L or less, or 1,000 mOsm/L or less.

When the specific gravity of the blood plasma separation material at 25°C is 1.040 or more and less than 1.050, the osmotic pressure of the osmotic pressure measurement solution is 330 mOsm/L or more, preferably 350 mOsm/L or more, more preferably 400 mOsm/L or more, and still more preferably 500 mOsm/L or more. When the osmotic pressure is the lower limit or more, the specific gravities of white blood cells and red blood cells can be effectively increased, and the contamination of white blood cells and red blood cells into blood plasma can be more effectively suppressed. Note that the upper limit of the osmotic pressure of the osmotic pressure measurement solution when the specific gravity of the blood plasma separation material at 25°C is 1.040 or more and less than 1.050 is not particularly limited. When the specific gravity of the blood plasma separation material at 25°C is 1.040 or more and less than 1.050, the osmotic pressure of the osmotic pressure measurement solution may be, for example, 1,500 mOsm/L or less, or 1,000 mOsm/L or less.

When the specific gravity of the blood plasma separation material at 25°C is 1.050 or more and less than 1.060, the osmotic pressure of the osmotic pressure measurement solution is 350 mOsm/L or more, preferably 380 mOsm/L or more, more preferably 450 mOsm/L or more, and still more preferably 500 mOsm/L or more. When the osmotic pressure is the lower limit or more, the specific gravities of white blood cells and red blood cells can be effectively increased, and the contamination of white blood cells and red blood cells into blood plasma can be more effectively suppressed. Note that the upper limit of the osmotic pressure of the osmotic pressure measurement solution when the specific gravity of the blood plasma separation material at 25°C is 1.050 or more and less than 1.060 is not particularly limited. When the specific gravity of the blood plasma separation material at 25°C is 1.050 or more and less than 1.060, the osmotic pressure of the osmotic pressure measurement solution may be, for example, 1,500 mOsm/L or less, or 1,000 mOsm/L or less.

When the specific gravity of the blood plasma separation material at 25°C is 1.060 or more and less than 1.070, the osmotic pressure of the osmotic pressure measurement solution is 500 mOsm/L or more, preferably 550 mOsm/L or more, more preferably 600 mOsm/L or more, and still more preferably 700 mOsm/L or more. When the osmotic pressure is the lower limit or more, the specific gravities of white blood cells and red blood cells can be effectively increased, and the contamination of white blood cells and red blood cells into blood plasma can be more effectively suppressed. Note that the upper limit of the osmotic pressure of the osmotic pressure measurement solution when the specific gravity of the blood plasma separation material at 25°C is 1.060 or more and less than 1.070 is not particularly limited. When the specific gravity of the blood plasma separation material at 25°C is 1.060 or more and less than 1.070, the osmotic pressure of the osmotic pressure measurement solution may be, for example, 1,000 mOsm/L or less, or 800 mOsm/L or less.

When the specific gravity of the blood plasma separation material at 25°C is 1.070 or more and 1.120 or less, the osmotic pressure of the osmotic pressure measurement solution is 650 mOsm/L or more, preferably 700 mOsm/L or more, and more preferably 800 mOsm/L or more. When the osmotic pressure is the lower limit or more, the specific gravities of white blood cells and red blood cells can be effectively increased, and the contamination of white blood cells and red blood cells into blood plasma can be more effectively suppressed. Note that the upper limit of the osmotic pressure of the osmotic pressure measurement solution when the specific gravity of the blood plasma separation material at 25°C is 1.070 or more and 1.120 or less is not particularly limited. When the specific gravity of the blood plasma separation material at 25°C is 1.070 or more and 1.120 or less, the osmotic pressure of the osmotic pressure measurement solution may be, for example, 1,500 mOsm/L or less, or 1,000 mOsm/L or less.

(Blood plasma separation material)

The blood collection container includes a blood plasma separation material contained in the blood collection container main body. The specific gravity of the blood plasma separation material at 25°C is 1.030 or more and 1.120 or less. As the blood plasma separation material, a conventionally known blood plasma separation material can be used. Examples of the blood plasma separation material include a blood plasma separation composition and a blood plasma separation jig. Since the blood plasma separation material is easily prepared, the blood plasma separation material is preferably the above-described blood plasma separation composition.

The specific gravity of the blood plasma separation material at 25°C may be 1.040 or more, may be 1.050 or more, may be 1.060 or more, may be more than 1.060, or may be 1.070 or more. The specific gravity of the blood plasma separation material at 25°C may be less than 1.070, may be less than 1.060, may be less than 1.050, or may be less than 1.040.

The storage location of the blood plasma separation material is not particularly limited as long as the location is in the blood collection container main body. The blood plasma separation material may be disposed on the bottom part of the blood collection container main body or may be disposed on its inner wall surface.

<Blood plasma separation composition>

The blood plasma separation composition is a composition that moves between a blood plasma layer and a blood cell layer during centrifugation to form a partition wall. In addition, the blood plasma separation composition is used for the purpose of preventing component migration between the blood plasma layer and the blood cell layer after centrifugation. The blood plasma separation composition preferably has thixotropy. The blood plasma separation composition may be contained in the bottom part of the blood collection container main body or may be disposed on the inner wall surface. From the viewpoint of more effectively exhibiting the effect of the present invention, preferably, the blood plasma separation composition is contained in the bottom part of the blood collection container main body.

As the blood plasma separation composition, a conventionally known blood plasma separation composition can be used.

The blood plasma separation composition preferably contains an organic component having fluidity at 25°C and an inorganic fine powder. One kind alone of the organic component having fluidity at 25°C and one kind alone of the inorganic fine powder may be used, and two or more kinds of the organic components and the inorganic components may be used in combination.

Organic component having fluidity at 25°C:
The phrase "having fluidity at 25°C" means that a viscosity at 25°C is 500 Pa•s or less.

The viscosity of the organic component at 25°C is preferably 30 Pa•s or more, more preferably 50 Pa•s or more, and preferably 200 Pa•s or less, more preferably 100 Pa•s or less. When the viscosity is the lower limit or more and the upper limit or less, the fluidity of the blood plasma separation composition is improved, and the strength of the partition wall can be improved.

The viscosity of the organic component at 25°C is measured using an E-type viscometer (e.g., "TVE-35" manufactured by Toki Sangyo Co., Ltd.) under the conditions of a temperature of 25°C and a shear rate of 1.0 seconds$^{-1}$.

Examples of the organic component include a resin, and a mixture of a resin and an organic compound such as a plasticizer. Therefore, the organic component preferably contains the resin, and more preferably contains the resin and the organic compound. When the organic component is a mixture of the resin and the organic compound, the organic component only has to have fluidity as the mixture (organic component), or the resin or the organic compound does not necessarily have to have fluidity. When the organic component is a mixture of the resin and the organic compound, the resin may be, for example, a resin that is solid at 25°C. One kind alone of the resin and one kind alone of the organic compound may be used, or two or more kinds of the resins and the organic components may be used in combination.

Examples of the resin include a petroleum resin, a cyclopentadiene-based resin, a polyester resin, a polyurethane resin, a (meth)acrylic-based resin, a silicone resin, an $\alpha$-olefin-fumaric acid ester copolymer, a copolymer of sebacic acid, 2,2-dimethyl-1,3-propanediol, and 1,2-propanediol, a polyether polyurethane-based resin, and a polyether polyester-based resin. One kind alone of the resin may be used, and two or more kinds of the resins may be used in combination.

The resin preferably contains a petroleum resin, a cyclopentadiene-based resin, a polyester resin, or a (meth)acrylic-based resin.

Examples of commercially available products of the petroleum resin include "Rigalite S5090" manufactured by Eastman Chemical Company.

Examples of the cyclopentadiene-based resin include a polymer of a cyclopentadiene-based monomer, a copolymer of a cyclopentadiene-based monomer and an aromatic monomer, and a dicyclopentadiene-based resin. The cyclopentadiene-based resin may be hydrogenated. The polymer of the cyclopentadiene-based monomer and the copolymer of the cyclopentadiene-based monomer and the aromatic monomer may be oligomers.

Examples of the cyclopentadiene-based monomer include cyclopentadiene, dicyclopentadiene, and alkyl-substituted derivatives of cyclopentadiene.

Examples of the aromatic monomer include styrene, methylstyrene, indene, and methylindene.

Examples of a commercially available product of the dicyclopentadiene resin include "Scoretz SU500" and" Scoretz SU90" manufactured by Colon.

Examples of the polyester resin include a polyalkylene terephthalate resin and a polyalkylene naphthalate resin. Examples of the polyalkylene terephthalate resin include polyethylene terephthalate, polybutylene terephthalate, and poly-1, 4-cyclohexanedimethylene terephthalate.

Examples of the polyurethane resin include a reaction product of a polyol compound and an isocyanate compound.

Examples of the (meth)acrylic-based resin include a resin obtained by polymerizing at least one (meth)acrylic acid ester monomer, and a resin obtained by polymerizing at least one (meth)acrylic acid ester monomer and a monomer other than the (meth)acrylic acid ester monomer.

Examples of the (meth)acrylic acid ester monomer include (meth)acrylic acid alkyl ester, (meth)acrylic acid polyalkylene glycol ester, (meth)acrylic acid alkoxyalkyl ester, (meth)acrylic acid hydroxyalkyl ester, (meth)acrylic acid glycidyl ester, (meth)acrylic acid dialkylaminoalkyl ester, (meth)acrylic acid benzyl ester, (meth)acrylic acid phenoxyalkyl ester, (meth)acrylic acid cyclohexyl ester, (meth)acrylic acid isobornyl ester, and (meth)acrylic acid alkoxysilyl alkyl ester having an alkyl group having 1 to 20 carbon numbers. One kind alone of the (meth)acrylic acid ester monomer may be used, or two or more kinds of the (meth) acrylic acid ester monomers may be used in combination.

Examples of the organic compound include benzene polycarboxylic acid alkyl ester derivatives. The organic compound is preferably a benzene polycarboxylic acid alkyl ester derivative. Therefore, the organic component is preferably a mixture of the resin and the benzene polycarboxylic acid alkyl ester derivative.

Examples of the benzene polycarboxylic acid alkyl ester derivative include phthalic acid esters, trimellitic acid esters, and pyromellitic acid esters. One kind alone of the benzene polycarboxylic acid alkyl ester derivative may be used, or two or more kinds of the benzene polycarboxylic acid alkyl ester derivatives may be used in combination.

Examples of the trimellitic acid ester include tri-n-octyl trimellitic acid, triisooctyl trimellitic acid, and triisodecyl trimellitic acid.

Examples of the pyromellitic acid ester include tetraisooctyl pyromellitic acid.

Examples of commercially available products of the trimellitic acid ester include "Monosizer W700" and" Monosizer W-750" manufactured by DIC Corporation, and "Sansosizer TOTM" and" Sansosizer TITM" manufactured by New Japan Chemical Co., Ltd.

Examples of commercially available products of the pyromellitic acid ester include "Monosizer W-7010" manufactured by DIC Corporation.

The benzene polycarboxylic acid alkyl ester derivative is preferably a phthalic acid ester, a trimellitic acid ester, or a pyromellitic acid ester, and more preferably a trimellitic acid ester.

Inorganic fine powder:

Examples of the inorganic fine powder include fine powder silica, titanium oxide powder, zinc oxide powder, alumina powder, glass fine powder, talc powder, kaolin powder, bentonite powder, titania powder, and zirconium powder.

When there is obtained a blood plasma separation composition having a specific gravity at 25°C of 1.05 or more (preferably a blood plasma separation composition having a specific gravity at 25°C of 1.050 or more), the inorganic fine powder more preferably contains fine powder silica and inorganic fine powder different from the fine powder silica. The inorganic fine powder different from the fine powder silica is preferably an inorganic fine powder having a specific gravity larger than a specific gravity of the fine powder silica, and more preferably an inorganic fine powder having a specific gravity of three or more, such as a zinc oxide powder, a titanium oxide powder, or an alumina powder.

From the viewpoint of more effectively exhibiting the effect of the present invention, the inorganic fine powder preferably contains fine powder silica.

Examples of the fine powder silica include natural silica and synthetic silica. Examples of the synthetic silica include hydrophilic silica and hydrophobic silica. Hydrophilic silica has an action of imparting thixotropy to the blood plasma separation composition and adjusting the specific gravity by hydrogen bonding between hydroxyl groups on the particle surface. On the other hand, hydrophobic silica has a smaller thixotropic imparting effect than hydrophilic silica.

From the viewpoint of maintaining both the specific gravity and the thixotropy of the blood plasma separation composition in a suitable range, the fine powder silica preferably contains hydrophilic silica, and more preferably contains hydrophilic silica and hydrophobic silica. The fine powder silica preferably contains at least hydrophilic silica.

The content of hydrophilic silica in 100 wt% of the blood plasma separation composition is preferably 0.01 wt% or more, more preferably 0.1 wt% or more, further preferably 0.3 wt% or more, and preferably 2.50 wt% or less, more preferably 2.00 wt% or less. When the content of the hydrophilic silica is the lower limit or more and the upper limit or less, both the specific gravity and the thixotropy of the blood plasma separation composition can be maintained in a more suitable range.

The mean particle size of the fine powder silica is not particularly limited. The mean particle size of the fine powder silica may be 1 nm or more, 10 nm or more, 500 nm or less, or 100 nm or less.

The mean particle size of the fine powder silica is a mean diameter measured on a volume basis, and is a value of a median diameter (D50) of 50%. The mean volume particle size (D50) can be measured by a laser diffraction/scattering method, an image analysis method, a Coulter method, a centrifugal sedimentation method, or the like. The mean volume particle size (D50) is preferably determined by measurement with a laser diffraction/scattering method or an image analysis method.

The blood plasma separation composition may contain components other than the above-described components as long as the effects of the present invention are not impaired. The blood plasma separation composition may contain, for example, an organic gelling agent, a thermoplastic elastomer, polyalkylene glycol, silicone oil, an auxiliary solvent, an antioxidant, a colorant, water, and the like as the other components. One kind alone of the other components may be used, and two or more kinds of these components may be used in combination.

The specific gravity of the blood plasma separation composition at 25°C is 1.030 or more and 1.120 or less.

The specific gravity of the blood plasma separation composition at 25°C is measured by sequentially dropping one drop of the blood plasma separation composition into saline at 25°C whose specific gravity is adjusted stepwise at intervals of 0.002, and by flotation and sedimentation in saline.

The viscosity of the blood plasma separation composition at 25°C is preferably 100 Pa•s or more, more preferably 150 Pa•s or more, and preferably 500 Pa•s or less, more preferably 400 Pa•s or less. When the viscosity is the lower limit or more and the upper limit or less, the effect of the present invention can be more effectively exhibited.

The viscosity of the blood plasma separation composition at 25°C is measured under the conditions using an E-type viscometer (e.g., "TVE-35" manufactured by Toki Sangyo Co., Ltd.) under the conditions of a temperature of 25°C and a shear rate of 1.0 seconds$^{-1}$.

<Blood plasma separation jig>

The blood plasma separation jig is a jig that moves between a blood plasma layer and a blood cell layer during centrifugation to form a partition wall. In addition, the blood plasma separation jig is used for the purpose of preventing component migration between the blood plasma layer and the blood cell layer.

As the blood plasma separation jig, a conventionally known blood plasma separation jig can be used. Examples of the blood plasma separation jig include a mechanical separator (blood plasma separation jig) described in WO 2010/132783 A1 and the like.

Examples of the material of the blood plasma separation jig include elastomer.

(Osmotic pressure regulator)

The blood collection container includes an osmotic pressure regulator contained in the blood collection container main body. As the osmotic pressure regulator, a conventionally known osmotic pressure regulator can be used. One kind alone of the osmotic pressure regulator may be used, or two or more kinds of the osmotic pressure regulators may be used in combination.

Examples of the osmotic pressure regulator include sodium chloride, potassium chloride, glucose, dihydroxyacetone, and sugar alcohols such as D-mannitol and D-sorbitol.

From the viewpoint of more effectively exhibiting the effect of the present invention, the osmotic pressure regulator is preferably sodium chloride or glucose.

The osmotic pressure regulator may be contained in a powder state or may be contained in a state of being dissolved in a liquid in the blood collection container main body. Note that the osmotic pressure regulator may be present in both a powder state and a state of being dissolved in a liquid in the blood collection container main body.

Examples of the liquid include water and alcohol.

In addition, preferably, the osmotic pressure regulator is disposed on an inner wall surface of the blood collection container main body or disposed on a surface of the blood plasma separation material. Note that the osmotic pressure regulator may be disposed on the inner wall surface of the blood collection container main body, may be disposed on the surface of the blood plasma separation material, or may be disposed on both the inner wall surface of the blood collection container main body and the surface of the blood plasma separation material.

When the osmotic pressure regulator is contained in a powder state, preferably, the osmotic pressure regulator in a powder state is attached on the inner wall surface of the blood collection container main body or disposed on the surface of the blood plasma separation material.

The amount of the osmotic pressure regulator contained in the blood collection container main body is not particularly limited as long as the osmotic pressure of the osmotic pressure measurement solution satisfies the above-described range.

(Anticoagulant)

The blood collection container includes an anticoagulant contained in the blood collection container main body. As the anticoagulant, a conventionally known anticoagulant can be used. One kind alone of the anticoagulant may be used, or two or more kinds of the anticoagulants may be used in combination.

Examples of the anticoagulant include heparin, ethylenediaminetetraacetic acid (EDTA), and citric acid.

The anticoagulant may be contained in a powder state or may be contained in a state of being dissolved in a liquid in the blood collection container main body. Note that the anticoagulant may be present in both a powder state and a state of being dissolved in a liquid in the blood collection container main body.

Examples of the liquid include water and alcohol.

In addition, preferably, the anticoagulant is disposed on an inner wall surface of the blood collection container main body or disposed on a surface of the blood plasma separation material. Note that the anticoagulant may be disposed on the inner wall surface of the blood collection container main body, may be disposed on the surface of the blood plasma separation material, or may be disposed on both the inner wall surface of the blood collection container main body and the surface of the blood plasma separation material.

When the anticoagulant is contained in a powder state, preferably, the anticoagulant in a powder state is attached on the inner wall surface of the blood collection container main body or disposed on the surface of the blood plasma separation material.

The amount of the anticoagulant contained in the blood collection container main body is not particularly limited as long as the effect of the present invention is not impaired.

(Blood collection container main body)

Although the shape of the blood collection container main body is not particularly limited, the shape is preferably a bottomed tubular container.

The material of the blood collection container main body is not particularly limited. Examples of the material of the blood collection container main body include thermoplastic resins such as polyethylene, polypropylene, polystyrene, polyethylene terephthalate, polymethyl methacrylate, or polyacrylonitrile; thermosetting resins such as unsaturated polyester resin, epoxy resin, or epoxyacrylate resin; modified natural resins such as cellulose acetate, cellulose propionate, ethyl cellulose, and ethyl chitin; silicate glass such as soda lime glass, phosphosilicate glass, and borosilicate glass; and glass such as silica glass. As the material of the blood collection container main body, one kind may be used alone, or two or more kinds may be used in combination.

(Plug)

The blood collection container preferably includes a plug. As the plug, a conventionally known plug can be used. The plug is preferably formed of a material or a shape that can be airtightly and liquid-tightly attached to the opening of the blood collection container main body. The plug is preferably formed such that a blood collection needle can pierce the plug.

Examples of the plug include a plug having a shape fitted to the opening of the blood collection container main body, a sheet-like seal plug, and the like.

The plug may include a plug body such as a rubber plug and a cap member made of plastic or the like. In this case, it is possible to suppress the risk that the blood comes into contact with the human body when the plug is pulled out from the opening of the blood collection container main body after the blood collection.

Examples of the material of the plug (or the plug main body) include synthetic resin, elastomer, rubber, and metal foil. Examples of the rubber include butyl rubber and halogenated butyl rubber. Examples of the metal foil include aluminum foil. From the viewpoint of enhancing the sealing property, the material of the plug is preferably butyl rubber. The plug (or the plug body) is preferably a butyl rubber plug.

(Other details of blood collection container)

The blood collection container is preferably a blood collection tube. The blood collection container main body is preferably a blood collection tube main body.

The blood collection container is used for separating blood plasma from blood. The blood collection container is particularly suitably used for detecting extracellular nucleic acid in blood. In the present invention, the contamination of white blood cells into blood plasma can be suppressed, and thus extracellular nucleic acid in the blood of a subject can be accurately detected by detecting extracellular nucleic acid in blood plasma. Examples of the extracellular nucleic acid include cell free DNA (cfDNA) and cell free RNA (cfRNA).

The blood collection container in which the osmotic pressure regulator and the anticoagulant are contained in a state of being dissolved in a liquid can be manufactured, for example, as follows.

The osmotic pressure regulator, the anticoagulant, and other components to be used as necessary are dissolved in a solvent such as water to obtain a mixed solution. The obtained mixed solution is added into the blood collection container main body. In addition, before or after the mixed solution is added, the blood plasma separation composition is contained in the blood collection container main body.

Note that it is possible to obtain the blood collection container in which the osmotic pressure regulator and the anticoagulant are disposed on the surface of the blood plasma separation composition in a powder state by volatilizing the solvent in the mixed solution or adding the osmotic pressure regulator, the anticoagulant, and the like in a powder state onto the surface of the blood plasma separation composition.

The blood collection container in which the osmotic pressure regulator and the anticoagulant are disposed in a powder state on an inner wall surface of the blood collection container main body can be manufactured, for example, as follows.

The osmotic pressure regulator, the anticoagulant, and other components to be used as necessary are dissolved in a solvent such as water to obtain a mixed solution. The mixed solution is applied to the inner wall surface of the blood collection container main body and dried. In addition, before or after applying the mixed solution, the blood plasma separation composition is contained in the blood collection container main body.

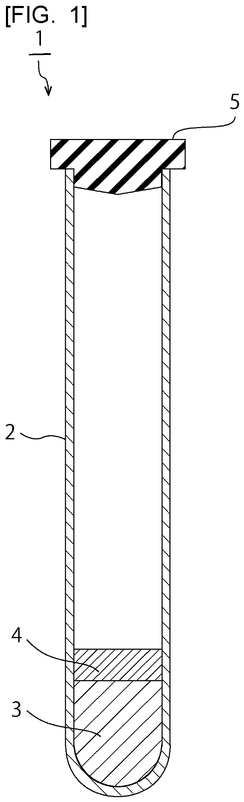

Fig. 1 is a front cross-sectional view of a blood collection container according to a first embodiment of the present invention.

A blood collection container 1 shown in Fig. 1 includes a blood collection container main body 2, a blood plasma separation composition 3, a mixed solution 4 containing the osmotic pressure regulator, the anticoagulant, and water, and a plug 5. The blood collection container body 2 has an opening at one end and a bottom closed at the other end. The blood plasma separation composition 3 is contained in the bottom part of the blood collection container main body 2. The plug 5 is inserted into an opening of the blood collection container main body 2.

The mixed solution 4 containing the osmotic pressure regulator, the anticoagulant and water is disposed on the surface of the blood plasma separation composition 3, more specifically, on the upper surface (surface on one end side) of the blood plasma separation composition 3. The mixed solution 4 is disposed on the surface of the blood plasma separation composition 3 when the blood collection container is in an upright state. The osmotic pressure regulator and the anticoagulant are contained in the blood collection container main body 2 in a state of being dissolved in a liquid.

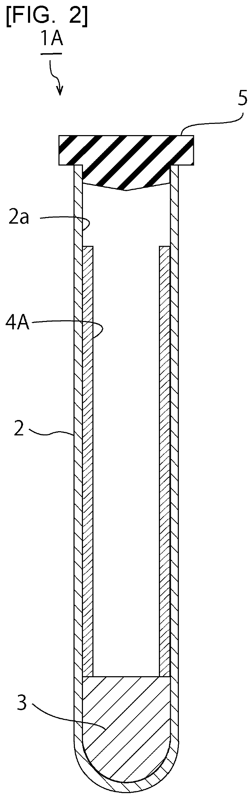

Fig. 2 is a front cross-sectional view of a blood collection container according to a second embodiment of the present invention.

A blood collection container 1A shown in Fig. 2 includes a blood collection container main body 2, a blood plasma separation composition 3, a mixed powder 4A of the osmotic pressure regulator and the anticoagulant, and a plug 5. The blood collection container body 2 has an opening at one end and a bottom closed at the other end. The blood plasma separation composition 3 is contained in the bottom part of the blood collection container main body 2. The plug 5 is inserted into an opening of the blood collection container main body 2.

The mixed powder 4A of the osmotic pressure regulator and the anticoagulant is disposed on the inner wall surface 2a of the blood collection container main body 2. The mixed powder 4A is attached to the inner wall surface 2a of the blood collection container main body 2. That is, the osmotic pressure regulator and the anticoagulant are attached on the inner wall surface 2a of the blood collection container main body 2. The osmotic pressure regulator and the anticoagulant are contained in the blood collection container main body 2 in a powder state. The mixed powder 4A is disposed on one end side from the blood plasma separation composition 3.

In the blood collection container according to the present invention, the blood plasma separation composition may be disposed on an inner wall surface of the blood collection container main body, and the mixed solution may be disposed at the bottom part of the blood collection container main body when the blood collection container is in an upright state. In the blood collection container according to the present invention, the blood plasma separation composition may be disposed on the inner wall surface of the blood collection container main body, and the mixed powder may be disposed on the inner wall surface of the blood collection container main body or the surface of the blood plasma separation composition. In the blood collection container according to the present invention, the blood plasma separation composition may be disposed on an inner wall surface of the blood collection container main body, and the mixed powder may be disposed on the bottom part of the blood collection container main body. In addition, the blood plasma separation jig may be used instead of the blood plasma separation composition.

The internal pressure of the blood collection container is not particularly limited. The blood collection container can also be used as a vacuum blood collection tube sealed with the sealing member after the inside is evacuated. When the blood collection container is the vacuum blood collection tube, it is possible to easily collect a certain amount of blood regardless of the technical difference between blood collectors.

From the viewpoint of preventing bacterial infection, the inside of the blood collection container is preferably sterilized in compliance with the standards of ISO and JIS.

(Method for separating blood plasma)

A method for separating blood plasma according to the present invention is a method for separating blood plasma using the above-described blood collection container, and includes the steps of: collecting blood in the blood collection container; and centrifuging the blood collection container into which the blood is collected.

The centrifugation conditions in the centrifugation step are not particularly limited as long as the partition wall can be formed with the blood plasma separation material to separate the blood plasma from blood cells. Examples of the centrifugal separation condition include a condition of performing centrifugal separation at 400 G or more and 4,000 G or less for 10 minutes or more and 120 minutes or less.

In the following, the present invention will be described in more detail with reference to examples. The present invention is not limited only to the examples below.

As materials of the blood plasma separation composition, materials below were prepared.

(Material of organic component having fluidity at 25°C)

(Meth)acrylic-based resin:
In the presence of an azo-based polymerization initiator, 2-ethylhexyl acrylate and butyl acrylate were radically polymerized by a solution polymerization method to obtain a (meth)acrylic acid ester-based polymer having fluidity at 25°C.

Other resins:

Petroleum resin ("Rigalite S5090" manufactured by Eastman Chemical Company)
Dicyclopentadiene resin 1 ("Scoretz SU500" manufactured by Colon)
Dicyclopentadiene resin 2 ("Scoretz SU90" manufactured by Colon)

Organic compound:
Trimellitic acid ester (Benzene polycarboxylic acid alkyl ester derivative, "Monosizer W700" manufactured by DIC Corporation)

(Inorganic fine powder)

Hydrophilic silica (fine powder silica, "200CF" manufactured by Nippon Aerosil Co., Ltd.)
Hydrophobic silica (fine powder silica, "R974" manufactured by Nippon Aerosil Co., Ltd.)
Titanium oxide powder ("A-100" manufactured by Ishihara Sangyo Co., Ltd.)

(Other components)

Silicone oil ("SF 8410" manufactured by Dow Corning Toray Co., Ltd.)
Organic gelling agent ("Gelol D" manufactured by New Japan Chemical Co., Ltd.)
1-Methyl-2-pyrrolidone (auxiliary solvent)

Preparation of blood plasma separation compositions A to I:
Blood plasma separation compositions A to I were prepared by mixing an organic component having fluidity at 25°C, an inorganic fine powder, and other components at blending ratios shown in Tables 1 and 2.

Preparation of blood plasma separation composition J:
Materials of an organic component having fluidity at 25°C described in Table 2 were blended, heated and dissolved at 130°C, and mixed to prepare an organic component having fluidity at 25°C. Subsequently, an organic component having fluidity at 25°C, an inorganic fine powder, and other components were mixed at a blending ratio described in Table 2 to prepare component J for blood plasma separation.

[Table 1]

| | | | Blood plasma separation composition A | Blood plasma separation composition B | Blood plasma separation composition C | Blood plasma separation composition D | Blood plasma separation composition E |
|---|---|---|---|---|---|---|---|
| Organic component having fluidity at 25°C | (Meth)acrylic-based resin | wt% | 99.14 | 97.60 | 96.20 | 94.90 | 93.70 |
| | Petroleum resin | wt% | - | - | - | - | - |
| | Dicyclopentadiene resin 1 | wt% | - | - | - | - | - |
| | Dicyclopentadiene resin 2 | wt% | - | - | - | - | - |
| | Trimellitic acid ester | wt% | - | - | - | - | - |
| Inorganic fine powder | Fine powder silica — Hydrophilic silica | wt% | 0.40 | 1.60 | 1.25 | 1.00 | 0.75 |
| | Fine powder silica — Hydrophobic silica | wt% | - | 0.70 | 1.95 | 2.20 | 2.45 |
| | Titanium oxide powder | wt% | - | - | 0.50 | 1.80 | 3.00 |
| Others | Silicone oil | wt% | 0.10 | 0.10 | 0.10 | 0.10 | 0.10 |
| | Organic gelling agent | wt% | 0.06 | - | - | - | - |
| | 1-Methyl-2-pyrrolidone | wt% | 0.30 | - | - | - | - |
| Total | | wt% | 100 | 100 | 100 | 100 | 100 |

[Table 2]

| | | | Blood plasma separation composition F | Blood plasma separation composition G | Blood plasma separation composition H | Blood plasma separation composition I | Blood plasma separation composition J |
|---|---|---|---|---|---|---|---|
| Organic component having fluidity at 25°C | (Meth)acrylic-based resin | wt% | 92.40 | 91.35 | 88.50 | 85.20 | - |
| | Petroleum resin | wt% | - | - | - | - | 14.50 |
| | Dicyclopentadiene resin 1 | wt% | - | | | - | 18.00 |
| | Dicyclopentadiene resin 2 | wt% | - | - | | - | 19.70 |
| | Trimellitic acid ester | wt% | - | - | - | - | 44.90 |
| Inorganic fine powder | Fine powder silica | Hydrophilic silica | wt% | 0.60 | 0.20 | 0.20 | 0.20 | 0.70 |
| | | Hydrophobic silica | wt% | 2.60 | 3.00 | 3.00 | 3.00 | 1.80 |
| | Titanium oxide powder | wt% | 4.30 | 5.35 | 8.20 | 11.50 | - |
| Others | Silicone oil | wt% | 0.10 | 0.10 | 0.10 | 0.10 | |
| | Organic gelling agent | wt% | - | - | - | - | 0.06 |
| | 1-Methyl-2-pyrrolidone | wt% | - | - | - | | 0.34 |
| Total | | wt% | 100 | 100 | 100 | 100 | 100 |

    (Osmotic pressure regulator)
    Sodium chloride
    Glucose

    (Anticoagulant)
    Ethylenediaminetetraacetic acid dipotassium salt dihydrate (EDTA 2K•2H2O)

(Example 1)

The osmotic pressure regulator and the anticoagulant were dissolved in water to obtain a mixed solution. The types and blending amounts of the blending components of the obtained mixed solution are shown in Table 3.
A PET bottomed tube (blood collection container main body) having a length of 100 mm and an inner diameter of an opening of 14 mm was prepared. The blood plasma separation composition A (1.0 g) was contained in the bottom part of the blood collection container main body. In addition, 1.0 mL of the obtained mixed solution was added onto the surface of the blood plasma separation composition A. The inside of the blood collection container was depressurized and sealed with a butyl rubber stopper. In this way, a blood collection container was prepared. In the obtained blood collection container, the osmotic pressure regulator and the anticoagulant are disposed on the surface of the blood plasma separation composition in a state of being dissolved in a liquid. In addition, the obtained blood collection container is a container for collecting 4 mL of blood.

(Examples 2 to 12 and comparative examples 2 to 5)

Blood collection containers were prepared in the same manner as in Example 1 except that the types and compositions of the blood plasma separation composition, the osmotic pressure regulator, and the anticoagulant were changed as shown in Tables 3 to 6.

(Comparative example 1)

The anticoagulant was dissolved in water to obtain a mixed solution. The types and blending amounts of the blending components of the obtained mixed solution are shown in Table 6.
A PET bottomed tube (blood collection container main body) having a length of 100 mm and an inner diameter of an opening of 14 mm was prepared. The blood plasma separation composition B (1.0 g) was contained in the bottom part of the blood collection container main body. In addition, 30 mg of the obtained mixed solution was applied to the inner wall surface of the blood collection container main body and dried. The inside of the blood collection container was depressurized and sealed with a butyl rubber stopper. In this way, a blood collection container was prepared. In the obtained blood collection container, the anticoagulant is disposed on a powder on an inner wall surface of the blood collection container main body. In addition, the obtained blood collection container is a container for collecting 4 mL of blood.

(Evaluation)

(1) Specific gravity of blood plasma separation composition at 25°C

One drop of the obtained blood plasma separation composition was sequentially dropped dropwise into saline at 25°C whose specific gravity was adjusted stepwise at intervals of 0.002, and the specific gravity was measured by flotation in saline.

(2) Osmotic pressure of osmotic pressure measurement solution

Physiological saline solution (4 mL) was added to the obtained blood collection container. After the addition, the mixture was mixed by inversion, and the osmotic pressure regulator and the anticoagulant were dissolved in physiological saline solution to obtain an osmotic pressure measurement solution. The osmotic pressure of the obtained osmotic pressure measurement solution was measured by a cryoscopic method using an osmometer ("OM-6060" manufactured by ARKRAY, Inc.).

(3) Partition wall formability

The blood of three persons was prepared, and the following steps were sequentially performed.
Blood collection step:
Blood (4 mL) was collected in the obtained blood collection container.
Centrifugation step:
The blood collection container was centrifuged at 1,500 G for 15 minutes.
Visual observation:
Before centrifugation, it was visually confirmed whether the blood plasma separation composition contained in the bottom part of the blood collection container main body moved between the blood cell layer and the blood plasma layer after centrifugation to form a partition wall satisfactorily. Specifically, the formability of the partition wall was evaluated by the following method.
Test (1): The blood collection container after centrifugation was visually observed. When the blood plasma separation composition was located between the blood cell layer and the blood plasma layer, it was determined as good. On the other hand, when the total amount of the blood plasma separation composition was located below the blood cell layer, it was determined as poor.
Test (2): The blood collection container after centrifugation was allowed to stand so that the bottom of the blood collection container faced upward at an angle of 90°. At this time, it was visually observed whether the blood cell component that was located on the bottom side of the blood collection container main body from the blood plasma separation composition moved to the open end side of the blood collection container main body from the blood plasma separation composition and the blood cell component was mixed with the blood plasma. When the blood cell component was not mixed in the blood plasma, the blood cell component was determined as good. On the other hand, when the blood cell component was mixed in the blood plasma, the blood cell component was determined as poor.

[Criteria for determining formability of partition wall]

∘: The determination results of Test (1) and Test (2) are both good.
×: The determination result of Test (1) or Test (2) is poor.

Note that in Comparative examples 4 and 5, since the result of the partition wall formability was poor, the following (4) the evaluation of the contamination of white blood cells in blood plasma was not performed.

(4) Contamination of white blood cells in blood plasma

The blood of three persons was prepared, and the following steps were sequentially performed.
Blood collection step:
Blood (4 mL) was collected in the obtained blood collection container.
Centrifugation step:
The blood collection container was centrifuged at 1,500 G for 15 minutes.
After centrifugation, the blood plasma located above the partition wall formed by the blood plasma separation composition was stirred by pipetting to suspend the remaining blood cells deposited on the partition wall formed by the blood plasma separation composition, and then collected.
The number of white blood cells in blood plasma was measured by analyzing the collected blood plasma using a multi-item automatic blood cell analyzer ("XE 5000" manufactured by Sysmex Corporation). In addition, for the prepared blood (whole blood sample), the number of white blood cells in the whole blood sample was measured in the similar manner. Note that the number of white blood cells is a mean value of results obtained by evaluating the prepared three blood samples.
The residual rate of white blood cells was calculated by the following equation.

$$\text{Residual rate of white blood cells (\%) = (the number of white blood cells contained in the separated blood plasma (cells))/(the number of white blood cells contained in the whole blood sample (cells)) × 100}$$

[Criteria for determining contamination of white blood cells in blood plasma]

∘: Residual rate of white blood cells is less than 10%
×: The residual rate of white blood cells is 10% or more.

The composition and results are shown in Tables 3 to 6 below.

[Table 3]

| | | | | Example 1 | Example 2 | Example 3 | Example 4 |
|---|---|---|---|---|---|---|---|
| Mixture | Anticoagulant | EDTA2K•2H$_2$O | wt% | 0.78 | 0.78 | 0.78 | 0.78 |
| | Osmotic pressure regulator | NaCl | wt% | 1.08 | 1.54 | 2.99 | 6.90 |
| | | Glucose | wt% | - | - | - | - |
| | Solvent | Water | wt% | 98.14 | 97.68 | 96.23 | 92.32 |
| | Total | | wt% | 100 | 100 | 100 | 100 |
| Evaluation | Blood plasma separation composition | Type | - | A | B | B | B |
| | | Specific gravity at 25°C | - | 1.035 | 1.045 | 1.045 | 1.045 |
| | Osmotic pressure of osmotic pressure measurement solution | | mOsm/L | 309 | 341 | 439 | 703 |
| | Partition wall formability | | Determination | ○ | ○ | ○ | ○ |
| | Contamination of white blood cells in blood plasma | Number of white blood cells | × 10$^4$ cells | 0 | 150 | 25 | 0 |
| | | Residual rate of white blood cells | % | 0 | 6 | 1 | 0 |
| | | | Determination | ○ | ○ | ○ | ○ |

[Table 4]

| | | | | Example 5 | Example 6 | Example 7 | Example 8 |
|---|---|---|---|---|---|---|---|
| Mixture | Anticoagulant | EDTA2K•2H$_2$O | wt% | 0.78 | 0.78 | 0.78 | 0.78 |
| | Osmotic pressure regulator | NaCl | wt% | 2.17 | 4.55 | 5.32 | 6.90 |
| | | Glucose | wt% | - | - | - | - |
| | Solvent | Water | wt% | 97.05 | 94.67 | 93.90 | 92.32 |
| | Total | | wt% | 100 | 100 | 100 | 100 |
| Evaluation | Blood plasma separation composition | Type | - | C | D | D | F |
| | | Specific gravity at 25°C | - | 1.055 | 1.065 | 1.065 | 1.085 |
| | Osmotic pressure of osmotic pressure measurement solution | | mOsm/L | 383 | 541 | 578 | 703 |
| | Partition wall formability | | Determination | ○ | ○ | ○ | ○ |
| | Contamination of white blood cells in blood plasma | Number of white blood cells | × 10$^4$ cells | 120 | 170 | 70 | 0 |
| | | Residual rate of white blood cells | % | 5 | 7 | 3 | 0 |
| | | | Determination | ○ | ○ | ○ | ○ |

[Table 5]

|  |  |  |  | Example 9 | Example 10 | Example 11 | Example 12 |
|---|---|---|---|---|---|---|---|
| Mixture | Anticoagulant | EDTA2K•2H$_2$O | wt% | 0.78 | 0.78 | 0.78 | 0.78 |
|  | Osmotic pressure regulator | NaCl | wt% | 6.90 | 9.25 | 6.90 | - |
|  |  | Glucose | wt% | - | - | - | 18.00 |
|  | Solvent | Water | wt% | 92.32 | 89.97 | 92.32 | 81.22 |
|  | Total | | wt% | 100 | 100 | 100 | 100 |
| Evaluation | Blood plasma separation composition | Type | - | G | H | J | B |
|  |  | Specific gravity at 25°C | - | 1.095 | 1.120 | 1.045 | 1.045 |
|  | Osmotic pressure of osmotic pressure measurement solution | | mOsm/L | 703 | 869 | 703 | 448 |
|  | Partition wall formability | | Determination | ○ | ○ | ○ | ○ |
|  | Contamination of white blood cells in blood plasma | Number of white blood cells | × 10$^4$ cells | 45 | 50 | 0 | 30 |
|  |  | Residual rate of white blood cells — % | % | 2 | 2 | 0 | 1 |
|  |  | Residual rate of white blood cells — Determination | Determination | ○ | ○ | ○ | ○ |

[Table 6]

| | | | | Comparative Example 1 | Comparative Example 2 | Comparative Example 3 | Comparative Example 4 | Comparative Example 5 | |
|---|---|---|---|---|---|---|---|---|---|
| Mixture | Anticoagulant | EDTA2K•2H$_2$O | wt% | 26.16 | 0.78 | 0.78 | 0.78 | 0.78 | |
| | Osmotic pressure regulator | NaCl | wt% | - | 1.23 | 6.10 | 6.10 | 9.25 | |
| | | Glucose | wt% | - | - | - | - | - | |
| | Solvent | Water | wt% | 73.84 | 97.99 | 93.12 | 93.12 | 89.97 | |
| | Total | | wt% | 100 | 100 | 100 | 100 | 100 | |
| Evaluation | Blood plasma separation composition | Type | - | B | C | E | H | I | White blood cell count in whole blood sample ($\times$ 10$^4$ cells) |
| | | Specific gravity at 25°C | - | 1.045 | 1.055 | 1.075 | 1.120 | 1.150 | |
| | Osmotic pressure of osmotic pressure measurement solution | | mOsm/L | 297 | 320 | 628 | 628 | 869 | |
| | Partition wall formability | | Determination | ○ | ○ | ○ | × | × | |
| | Contamination of white blood cells in blood plasma | Number of white blood cells | $\times$ 10$^4$ cells | 250 | 480 | 230 | - | - | 2400 |
| | | Residual rate of white blood cells | % | 10 | 20 | 10 | - | | - |
| | | | Determination | × | × | × | - | - | |

EP 4 071 470 A1

**EXPLANATION OF SYMBOLS**

1, 1A: Blood collection container
2: Blood collection container main body
2a: Inner wall surface
3: Blood plasma separation composition
4: Mixture
4A: Mixed powder
5: Plug

**Claims**

1. A blood collection container into which a predetermined amount of blood is collected, the blood collection container comprising:

    a blood collection container main body;
    a blood plasma separation material contained in the blood collection container main body;
    an osmotic pressure regulator contained in the blood collection container main body; and
    an anticoagulant contained in the blood collection container main body,
    a specific gravity of the blood plasma separation material at 25°C being 1.030 or more and 1.120 or less, and
    when an osmotic pressure measurement solution is obtained by dissolving the osmotic pressure regulator and the anticoagulant contained in the blood collection container main body with a physiological saline solution in an amount equivalent to the predetermined amount of blood collected in the blood collection container,
    when the specific gravity of the blood plasma separation material at 25°C is 1.030 or more and less than 1.040, an osmotic pressure of the osmotic pressure measurement solution being 300 mOsm/L or more,
    when the specific gravity of the blood plasma separation material at 25°C is 1.040 or more and less than 1.050, the osmotic pressure of the osmotic pressure measurement solution being 330 mOsm/L or more,
    when the specific gravity of the blood plasma separation material at 25°C is 1.050 or more and less than 1.060, the osmotic pressure of the osmotic pressure measurement solution being 350 mOsm/L or more,
    when the specific gravity of the blood plasma separation material at 25°C is 1.060 or more and less than 1.070, the osmotic pressure of the osmotic pressure measurement solution being 500 mOsm/L or more, and
    when the specific gravity of the blood plasma separation material at 25°C is 1.070 or more and 1.120 or less, the osmotic pressure of the osmotic pressure measurement solution being 650 mOsm/L or more.

2. The blood collection container according to claim 1, wherein the blood plasma separation material is a blood plasma separation composition.

3. The blood collection container according to claim 2, wherein

    the blood plasma separation composition contains an organic component having fluidity at 25°C and an inorganic fine powder,
    the organic component contains a resin, and
    the inorganic fine powder contains fine powder silica.

4. The blood collection container according to claim 3, wherein the fine powder silica comprises hydrophilic silica.

5. The blood collection container according to claim 4, wherein a content of the hydrophilic silica is 0.01 wt% or more and 2.50 wt% or less in 100 wt% of the blood plasma separation composition.

6. The blood collection container according to any one of claims 3 to 5, wherein the fine powder silica comprises hydrophilic silica and hydrophobic silica.

7. The blood collection container according to any one of claims 3 to 6, wherein when the specific gravity of the blood plasma separation composition at 25°C is 1.05 or more, the inorganic fine powder contains an inorganic fine powder having a specific gravity larger than a specific gravity of the fine powder silica.

8. The blood collection container according to any one of claims 3 to 7, wherein the resin comprises a petroleum resin, a cyclopentadiene-based resin, a polyester resin, or a (meth)acrylic-based resin.

9. The blood collection container according to any one of claims 1 to 8, wherein

the osmotic pressure regulator is contained in the blood collection container main body in a powder state or in a state of being dissolved in a liquid, and
the anticoagulant is contained in the blood collection container main body in a powder state or in a state of being dissolved in a liquid.

10. The blood collection container according to any one of claims 1 to 9, wherein

the osmotic pressure regulator is disposed on an inner wall surface of the blood collection container main body or disposed on a surface of the blood plasma separation material, and
the anticoagulant is disposed on an inner wall surface of the blood collection container main body or disposed on a surface of the blood plasma separation material.

11. The blood collection container according to any one of claims 1 to 10, wherein the osmotic pressure regulator is sodium chloride or glucose.

12. The blood collection container according to any one of claims 1 to 11, wherein the blood collection container is used for detecting extracellular nucleic acid in blood.

13. A method for separating blood plasma using the blood collection container according to any one of claims 1 to 12, the method comprising:

collecting blood into the blood collection container; and
centrifuging the blood collection container into which the blood is collected.

[FIG. 1]

[FIG. 2]

| | | |
|---|---|---|
| **INTERNATIONAL SEARCH REPORT** | International application No. | |
| | PCT/JP2020/044835 | |

**A. CLASSIFICATION OF SUBJECT MATTER**
G01N 33/48(2006.01)i
FI: G01N33/48 H; G01N33/48 E

According to International Patent Classification (IPC) or to both national classification and IPC

**B. FIELDS SEARCHED**

Minimum documentation searched (classification system followed by classification symbols)
G01N33/48

Documentation searched other than minimum documentation to the extent that such documents are included in the fields searched
| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2019/131613 A1 (SEKISUI MEDICAL CO., LTD.) 04 July 2019 (2019-07-04) claims | 1–13 |
| A | WO 2010/053180 A1 (HITACHI CHEMICAL INDUSTRY CO., LTD.) 14 May 2010 (2010-05-14) claims, paragraph [0033] | 1–13 |
| A | JP 06-201682 A (NIPPON PAINT CO., LTD.) 22 July 1994 (1994-07-22) claim 1, paragraphs [0045]-[0046] | 1–13 |
| A | JP 08-143461 A (BECTON, DICKINSON AND COMPANY) 04 June 1996 (1996-06-04) fig. 2 | 1–13 |

☐ Further documents are listed in the continuation of Box C.　　☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09 February 2021 (09.02.2021) | 02 March 2021 (02.03.2021) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**
Information on patent family members

International application No.

PCT/JP2020/044835

| Patent Documents referred in the Report | Publication Date | Patent Family | Publication Date |
|---|---|---|---|
| WO 2019/131613 A1 | 04 Jul. 2019 | CN 110753843 A | |
| WO 2010/053180 A1 | 14 May 2010 | US 2011/0250105 A1 paragraph [0074] EP 2360470 A1 CN 102209895 A TW 201026307 A | |
| JP 06-201682 A | 22 Jul. 1994 | US 5438000 A example 1 EP 597690 A2 CA 2102993 A1 | |
| JP 08-143461 A | 04 Jun. 1996 | US 5663285 A fig. 2 US 5807970 A EP 705882 A2 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010053180 A1

- WO 2010132783 A1